(19) Europäisches Patentamt
European Patent Office
Office européen des brevets

(11) **EP 3 303 557 B1**

(12) **EUROPEAN PATENT SPECIFICATION**

(45) Date of publication and mention of the grant of the patent:
**05.05.2021 Bulletin 2021/18**

(21) Application number: **16727348.1**

(22) Date of filing: **25.05.2016**

(51) Int Cl.:
**C12N 5/00** (2006.01)

(86) International application number:
**PCT/EP2016/061763**

(87) International publication number:
**WO 2016/193083 (08.12.2016 Gazette 2016/49)**

(54) **SMALL SCALE CULTIVATION METHOD FOR SUSPENSION CELLS**

KULTIVIERUNGSVERFAHREN IN KLEINEM MASSSTAB FÜR SUSPENSIONSZELLEN

PROCÉDÉ DE CULTURE À PETITE ÉCHELLE DE CELLULES EN SUSPENSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2015 LU 92727**
**19.01.2016 EP 16151811**

(43) Date of publication of application:
**11.04.2018 Bulletin 2018/15**

(73) Proprietor: **Glycotope GmbH**
**13125 Berlin (DE)**

(72) Inventors:
• **GOLETZ, Steffen**
**13125 Berlin (DE)**
• **STAHN, Rainer**
**13125 Berlin (DE)**
• **KREYE, Steffen**
**13125 Berlin (DE)**

(74) Representative: **Hemsath, Lars et al**
**König-Szynka-Tilmann-von Renesse**
**Patentanwälte Partnerschaft mbB**
**Mönchenwerther Straße 11**
**40545 Düsseldorf (DE)**

(56) References cited:
**EP-A2- 0 599 651          WO-A1-2012/085162**
**US-A1- 2004 185 534**

• **STEVEN M WOODSIDE ET AL: "Mammalian cell retention devices for stirred perfusion bioreactors", CYTOTECHNOLOGY, KLUWER ACADEMIC PUBLISHERS, DO, vol. 28, no. 1-3, 1 September 1998 (1998-09-01), pages 163-175, XP019236582, ISSN: 1573-0778, DOI: 10.1023/A:1008050202561**
• **Nicole A. Bleckwenn ET AL: "Large-Scale Cell Culture" In: "Current Protocols in Immunology", 1 January 2004 (2004-01-01), John Wiley & Sons, Inc., Hoboken, NJ, USA, XP055041131, ISBN: 978-0-47-114273-7 DOI: 10.1002/0471142735.ima01us59, page 10**
• **MORAN E B ET AL: "A systematic approach to the validation of process control parameters for monoclonal antibody production in fed-batch culture of a murine myeloma", BIOTECHNOLOGY AND BIOENGINEERING, WILEY & SONS, HOBOKEN, NJ, US, vol. 69, no. 3, 5 August 2000 (2000-08-05) , pages 242-255, XP002409406, ISSN: 0006-3592, DOI: 10.1002/1097-0290(20000805)69:3<242::AID-B IT2>3.0.CO;2-I**
• **CHU L ET AL: "Industrial choices for protein production by large-scale cell culture", CURRENT OPINION IN BIOTECHNOLOGY, LONDON, GB, vol. 12, 1 January 2001 (2001-01-01), pages 180-187, XP003014088, ISSN: 0958-1669, DOI: 10.1016/S0958-1669(00)00197-X**
• **FENG LI ET AL: "Cell culture processes for monoclonal antibody production", MABS, vol. 2, no. 5, 1 September 2010 (2010-09-01), pages 466-479, XP055166177, ISSN: 1942-0862, DOI: 10.4161/mabs.2.5.12720**

## Description

### FIELD OF THE INVENTION

The present invention pertains to small scale cell culture systems which simulate large scale perfusion cultures of suspension cells. The present invention in particular provides methods for cultivating cells wherein cell sedimentation is used to separate cells from culture medium for replacing part of the culture medium, wherein the method is used for simulating large scale cell cultures and for screening culturing conditions and cell clones for use in large scale cell cultures.

### BACKGROUND OF THE INVENTION

Recombinant protein production is a major aspect of the biotechnical industry of today. It is gaining more and more importance as the number of applications requiring high amounts of high-quality proteins increase on the market. Food production and in particular pharmacology are two main areas where the need for recombinant proteins steadily increases. Higher production efficiencies and consequently lower costs of the final product are needed for obtaining a commercially viable process.

However, at the same time a high product quality and compatibility with human applications is essential. More and more applications required recombinant production of the proteins in eukaryotic cells, in particular in higher eukaryotic cells. Especially proteins carrying post-translational modifications such a glycosylation (glycoproteins) significantly differ when expressing them in prokaryotic cell systems such as *E. coli* or eukaryotic cell systems such as in particular human cell lines. These differences in many cases markedly affect the biological activity as well as the immunogenicity of the produced proteins. However, many expression systems using higher eukaryotic cell lines suffer from a rather low expression rate of the desired protein, resulting in low yields and high costs of the recombinant protein.

For improving the yield and quality of recombinant proteins produced in eukaryotic cells, including the characteristics of their post-translational modifications, a multitude of culturing conditions are optimized and a large number of cell clones are tested. It is highly expensive and cumbersome to do this screening process in large scale cell cultures which are used for the later production of the recombinant protein. Therefore, such screening processes are often performed in small scale cultures. However, such small scale cultures often significantly differ in their behavior from the large scale cultures they shall simulate. When transferring into large scale, the culturing conditions and cell clones established in the small scale culture may prove to be less optimal.

Reliable down-scale models are crucial for efficient and fast bioprocess development. Key operations like media or process optimization and clone screening are usually not performed under process conditions due to several reasons like high cost of operation or low throughput. A major task for bioprocessing is to find appropriate down scale models which represent the larger scale as best as possible. An applicable down scale system must primarily feature satisfactory comparability of key parameters to the large scale and medium to high throughput.

The advanced micro bioreactor (ambr) system represents such a down scale tool with superior reproducibility to larger scale bioreactor (Hsu et al. (2012) Cytotechnology 64:667-678). The ambr system consists of single use vessels with a working volume of 10 to 15 ml, a liquid handling robot and monitor measuring oxygen saturation (DO) and pH online. The throughput is limited to 24 or 48 vessels depending on device equipment. The ambr system is widely applied as scale down model for batch and fed-batch cultivations. Perfusion culture, however, has not been available. The 1 l bench top bioreactor is considered to be the smallest scale down unit for perfusion culture (Shimoni et al. (2014) BioProcess Int 12:54-61). The obstacle for developing smaller scale perfusion system is the scale down of the cell retention device. Typical cell retention devices are discussed in Voisard et al. (2003) Biotechol Bioeng 82:751-765, and mechanisms based on filtration or centrifugation require too much dead volume to work efficient with small volume bioreactor. At the beginning of perfusion cell culture sedimentation was explored as cell separation method and further development let to external devices such inclined settlers. While being robust and causing little shear stress, cells reside outside of the bioreactor facing suboptimal conditions like lower temperature or insufficient oxygen supply (Voisard et al. (2003) Biotechol Bioeng 82:751-765).

WO 2012/085162 A1 describes different methods for small scale cell culture under different stirring and perfusion conditions and scale up experiments. EP 0 599 651 A2 describes a bioreactor for perfusion culture wherein the medium which is removed from the culture is substantially free of cells and wherein the cells settle by sedimentation. Woodside et al. (1998) Cytotechnology 28:163-175 describe different possibilities of cell retention in stirred perfusiopn bioreactors for perfusion or fed-batch cell culture, including sedimentation of cells. Bleckwenn et al. (2004) Current Protocols in Immunology, Supplement 59, A.1U.1-A.1U.44 reviews different methods for large scale cell culture. None of these documents describes a method for simulating a large scale perfusion cell culture using a small scale cell culture wherein cell retention for culture medium exchange is achieved by cell sedimentation after stop of stirring.

Therefore, there is a need in the art to provide methods for efficiently simulating large scale cell cultures in small scale for screening purposes, especially when using eukaryotic expression cell lines.

## SUMMARY OF THE INVENTION

As demonstrated by the present invention, perfusion cultures of eukaryotic cells in suspension can efficiently be simulated in small scale using a stirred cell culture wherein after certain time intervals a part of the medium is exchanged for fresh medium. Retention of the cells in the culture is achieved by sedimentation of the cells prior to removal of the culture medium. Cell concentration in the culture, cell viability and in particular the glycosylation pattern of the produced glycoprotein are highly similar between the small scale cell culture and the large scale perfusion culture it simulates.

Therefore, the present invention provides in a first aspect a method for simulating a large scale perfusion culture of cells in suspension, comprising

(a) providing a small scale cell culture having a culture volume of 10% or less of the large scale perfusion culture;

(b) cultivating cells in the small scale cell culture using one or more culturing conditions of the large scale perfusion culture to be simulated, wherein the small scale cell culture is stirred;

(c) stopping the stirring of the small scale cell culture;

(d) removing a part of the culture medium of the small scale cell culture from the top part of the cell culture after at least partial sedimentation of the cells in the small scale cell culture; and

(e) adding fresh culture medium to the small scale cell culture;

wherein the cells are eukaryotic cells which grow in suspension.

In a second aspects, the present invention provides a method for screening a culturing condition and/or a cell clone for a large scale perfusion culture of cells in suspension, comprising

(a) providing small scale cell cultures having a culture volume of 10% or less of the large scale perfusion culture;

(b) cultivating cells in the small scale cell cultures, wherein the small scale cell cultures are stirred;

(c) stopping the stirring of the small scale cell cultures;

(d) removing a part of the culture medium of the small scale cell cultures from the top part of the cell culture after at least partial sedimentation of the cells in the small scale cell cultures;

(e) adding fresh culture medium to the small scale cell cultures; and

(f) selecting the screened for culturing condition and/or cell clone which provides for a desired property and/or characteristic of the large scale perfusion culture;

wherein the individual small scale cell cultures differ in the culturing condition and/or the cell clone to be screened for; and wherein the cells are eukaryotic cells which grow in suspension.

Other objects, features, advantages and aspects of the present invention will become apparent to those skilled in the art from the following description and appended claims.

## DETAILED DESCRIPTION OF THE INVENTION

The present invention describes methods for cultivating cells in suspension in small scale. These cultivation methods can be used to simulate large scale perfusion cultures of cells in suspension. Furthermore, screening methods for determination of suitable culturing conditions and cell clones can be performed using said cultivation methods.

A method for cultivating cells in suspension may comprise

(a) providing a cell culture;

(b) cultivating cells in a cell culture, wherein the cell culture is stirred;

(c) stopping the stirring of the cell culture;

(d) removing a part of the culture medium of the cell culture after at least partial sedimentation of the cells in the cell culture; and

(e) adding fresh culture medium to the cell culture.

The cell culture provided in step (a) is a suspension cell culture which preferably is provided in a bioreactor equipped with a means for stirring the cell culture. The means for stirring the cell culture may in particular be an impeller such as a pitched blade impeller. The bioreactor may further be equipped with means to supply the cell culture with gas and/or liquid, and with means to measure one or more culturing conditions of the cell culture. Said culturing conditions may include the temperature, pH value, oxygen concentration, nutrient concentration and carbon dioxide concentration of the cell culture.

For automated operation of the cell culture, the bioreactor is further equipped with a control unit which is capable of controlling the supply of oxygen and/or nutrients, the temperature and/or the pH value of the cell culture. The control unit may automatically control one or more of these parameters based on the values of the one or more culturing conditions measured in the cell culture. Desired values or ranges for each culturing condition may be preset by the operator. A suitable bioreactor for use in the methods is a microbioreactor of the Advanced Microbioreactor (AMBR) series TAB Biosystems/Sartorius, in particular the AMBR24 or AMBR15.

In certain embodiments, a small scale cell culture is used. According to the invention, small scale cell cultures in particular have a culture volume of 250 ml or less, in particular 100 ml or less, 50 ml or less, 25 ml or less or 12.5 ml or less. The volume of a small scale cell culture especially is in the range of from 1 ml to 200 ml, in particular from 2 ml to 150 ml, from 3 ml to 100 ml, from 5 ml to 75 ml, from7 ml to 50 ml, from 8 ml to 30 ml, or from 9 ml to 20 ml. In specific embodiments, the small scale cell culture has a volume of from 10 ml to 15 ml, in particular about 12 ml.

The cells in the cell culture in particular are suspension cells, i.e. cells that are able to survive and/or proliferate in suspension. In particular, the cells do not need to be attached to any solid surface in order to survive and/or proliferate. The cells are eukaryotic cells. In certain embodiments, the cells are immortalized human blood cells such as immortalized human cells of myeloid leukemia origin. Specific human cells of myeloid leukemia origin are K562 cells and cells derived therefrom, such as NM-F9, NM-D4, NM-H9D8, NM-H9D8-E6, NM H9D8-E6Q12, GT-2X, GT-5s and cells derived therefrom. K562 is a human myeloid leukemia cell line present in the American Type Culture Collection (ATCC CCL-243). The remaining cell lines are derived from K562 cells and have been selected for specific glycosylation features. Cell lines derived from K562 can be cultivated and maintained under the well-known conditions suitable for K562. All these cell lines except for K562 cells were deposited according to the Budapest treaty. Information on the deposition can be found at the end of the specification. In another embodiment, the cells are CHO cells.

In specific embodiments, the cells in the cell culture produce a biomolecule of interest. The cells may produce the biomolecule of interest recombinantly. In certain embodiments, the biomolecule of interest is a protein, in particular a glycoprotein such as an antibody.

In step (b), the cells are cultivated in the cell culture. The culturing conditions of the cell culture are generally chosen so as to accommodate the cells to be cultivated. In certain embodiments, the cells are cultivated at a temperature of about 37°C, in particular in the range of from 33°C to 40°C, especially from 35°C to 38°C. Furthermore, in certain embodiments the cells are cultivated at a pH value of about 7.1, in particular a pH value in the range of from 6.5 to 7.7, especially from 6.7 to 7.5, particularly from 6.9 to 7.3. The cell density in the cell culture may be at least $1 \times 10^4$ cells/ml, in particular at least $5 \times 10^4$ cells/ml or at least $1 \times 10^5$ cells/ml. The cell density is especially in the range of from about $1 \times 10^5$ cells/ml to about $5 \times 10^5$ cells/ml, particularly from about $2 \times 10^5$ cells/ml to about $3 \times 10^5$ cells/ml. In specific embodiments, the oxygen concentration in the culture medium is at least 10%, in particular at least 20%, especially in the range of from 10% to 80%, particularly from 20% to 60%.

In certain embodiments, the pH value and/or the oxygen concentration in the culture medium are controlled during cultivation of the cells. Controlling of the pH value may be performed by adding carbon dioxide or NaOH if the pH value is outside of a desired range. Carbon dioxide is added to decrease the pH value, i.e. in cases where the pH value is too high, and NaOH is added to increase the pH value, i.e. in cases where the pH value is too low. Controlling of the oxygen concentration may be performed by controlling the amount of oxygen passed into the culture medium. In certain embodiments, the cell culture is supplied with oxygen.

During cultivation of the cells, the cell culture is stirred. In specific embodiments, the cell culture is stirred with an impeller. Any impeller suitable for stirring cell cultures may be used, especially pitched blade impellers. The stirring speed should be adjusted to the used bioreactor and the cultivated cells. For example, in a small scale cell culture having, for example, a volume of about 5 ml to 50 ml, the stirring speed of the impeller is in the range of from about 500 rpm to about 2000 rpm, such as between 750 rpm and 1500 rpm.

In step (c), the stirring of the cell culture is stopped. In certain embodiments, the cells are cultivated under stirring in step (b) for at least 1 h before the stirring is stopped in step (c). In particular, the cells are cultivated under stirring for at least 1.5 h, at least 2 h, at least 2.5 h or at least 3 h. In certain embodiments, the cells are cultivated under stirring for at least 8 h, at least 10 h, at least 11 h or at least 12 h. After stopping the stirring, the cells are allowed to sediment. Sedimentation of the cells may take place for at least 30 min before removing a part of the culture medium in step (d). In certain embodiments, sedimentation takes place for at least 35 min, in particular for at least 40 min or at least 45 min. Especially, the cells are allowed to sediment for a time of from 30 min to 45 min, in particular about 40 min. In certain embodiments, the time of sedimentation is adjusted to the behavior of the cultured cells. In particular, the sedimentation time is long enough to allow at least 75%, in particular at least 90%, at least 95% or at least 99% of the cells in the cell culture to settle to the lower 75% of the culture container, in particular to the lower half of the culture container, especially to the ground of the culture container. In specific embodiments, sedimentation of the cells occurs without the addition or presence of any means or substances which induce or enhance sedimentation of the cells.

In specific embodiments, the control of the pH value and/or the control of the oxygen concentration are stopped before stopping the stirring of the cell culture in step (c). In particular, the control of both the pH value and the oxygen concentration are stopped. The control of the pH value and/or the control of the oxygen concentration may be stopped at least 5 min, especially at least 10 min or at least 15 min before stopping the stirring of the cell culture in step (c).

In another embodiment, sedimentation of the cells after stopping the stirring in step (c) takes place for 35 min or less. Using shorter sedimentation times, not all cells in the cell culture will be settled to the ground at the end of the sedimentation. When removing part of the culture medium in step (d), in this embodiment the removed culture medium will contain a significant amount of cells. Hence, by removing part of the culture medium cell bleeding is achieved. Thereby, the cell density is controlled and these cells are kept in the proliferation phase. In these embodiments, in step (d) also a part of the cells in the cells culture may be removed to effect cell bleeding. In particular, the sedimentation time is adjusted so that from about 1% to about 25%, in particular from about 2% to about 20%, about 5% to about 15% are about 7.5% to about 12.5% of the cells in the cell culture are removed with the culture medium removed in step (d). In specific embodiments, sedimentation of the cells after stopping the stirring in step (c) takes place for 30 min or less, 25 min or less or 20 min or less before removing a part of the culture medium in step (d). In particular, sedimentation of the cells after stopping the stirring in step (c) takes place for about 15 min to about 35 min, especially for about 20 min to about 30 min before removing a part of the culture medium in step (d).

In step (d), a part of the culture medium of the cell culture is removed. In certain embodiments, from about 10% to about 75% of the culture medium is removed. In particular, from about 50% to about 50%, especially from about 20% to about 30% and particularly about 25% of the culture medium is removed in step (d). The culture medium is removed in step (d) from the top part of the cell culture. In embodiments where no cell bleeding is intended, the culture medium removed in step (d) in particular comprises less than 10%, especially less than 5%, less than 2.5% or particularly less than 1% of the cells in the cell culture.

In step (e), fresh culture medium is added to the cell culture. In specific embodiments, the amount of fresh culture medium added in step (e) is in essence identical to the amount of culture medium removed in step (d). In particular, the amount of culture medium added in step (e) is in the range of from 75% to 125%, especially from 90% to 110% or from 95% to 105%, particularly about 100% of the amount of culture medium removed in step (d).

In specific embodiments, steps (b) to (e) are repeated one or more times. In particular, the cells are cultivated for a certain time and after specific time intervals, the stirring is stopped, the cells are allowed to sediment, a part of the culture medium is removed, fresh culture medium is added and the stirring is started again. Then this cycle comprising steps (b) to (e) begins again. In certain embodiments, after step (e) the cells are cultivated under stirring for at least 1 h before the stirring is stopped in step (c) of the next cycle. In particular, the cells are cultivated under stirring for at least 1.5 h, at least 2 h, at least 2.5 h or at least 3 h. In certain embodiments, the cells are cultivated under stirring for at least 8 h, at least 10 h, at least 11 h or at least 12 h. The methods described herein may include at least one cycle per day, in particular at least 2, at least 3 or at least 4 cycles per day, up to, for example, 8 cycles per day.

In a first aspect, the present invention provides a method for simulating a large scale perfusion culture of cells in suspension, comprising

(a) providing a small scale cell culture having a culture volume of 10% or less of the large scale perfusion culture;

(b) cultivating cells in the small scale cell culture using one or more culturing conditions of the large scale perfusion culture to be simulated, wherein the small scale cell culture is stirred;

(c) stopping the stirring of the small scale cell culture;

(d) removing a part of the culture medium of the small scale cell culture from the top part of the cell culture after at least partial sedimentation of the cells in the small scale cell culture; and

(e) adding fresh culture medium to the small scale cell culture;

wherein the cells are eukaryotic cells which grow in suspension.

The one or more culturing conditions of the large scale perfusion culture to be simulated which are used for cultivating cells in the small scale cell culture may in particular be selected from the group consisting of culture medium or specific culture medium ingredients, temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium.

The small scale cell culture does not simulate the culture volume of the large scale perfusion culture. In specific embodiments, the small cell culture does not simulate method of separating the cells from the culture medium to be removed used in the large scale perfusion culture.

Simulation of a large scale perfusion culture by a small scale cell culture in particular means that certain properties and characteristics observed in the small scale cell culture will similarly be present in the large scale perfusion culture. Thereby, specific culturing conditions and/or cell clones can be tested in the small scale cell culture. If these culturing conditions and/or cell clones prove to be advantageous, then they can be used in the large scale culture. In certain embodiments, the large scale perfusion culture is simulated by the small scale cell culture with respect to one or more properties and/or characteristics selected from the group consisting of cell growth, cell viability and cell density In embodiments where the cells produce a biomolecule of interest, in particular a glycoprotein, the properties and/or characteristics of the large scale perfusion culture which are simulated by the small scale cell culture may further include the yield of the biomolecule of interest, the productivity of the cells, the stability of the production of the biomolecule of interest and the quality of the biomolecule of interest, including in particular one or more glycosylation characteristics of the biomolecule in case it is a glycoprotein. The glycosylation features especially include the relative amount of glycosylation, i.e. what percentage of the glycosylation sites in the glycoprotein are occupied by a glycan structure; the relative amount of the type of glycan structure, i.e. what percentage of all glycan structures attached to the glycoprotein are glycan structures of a specific type, such as complex-type N-glycans; the relative amount of the different monosaccharides, i.e. what percentage of all monosaccharides in the glycan structures attached to the glycoprotein are of a specific kind of monosaccharide, such as. and galactose, fucose, sialic acid, N-acetylglucosamine and mannose; and the relative amount of specific glycosylation motifs, i.e. what percentage of all glycan structures exhibit a specific glycosylation motif, such as core fucose, bisecting N-acetylglucosamine, at least one galactose residue, at least two galactose residues, at least one sialic acid residue, at least two sialic acid residues and monoantennary, diantennary, triantennary and tetraantennary glycans.

In a certain embodiment, the method for simulating a large scale perfusion culture comprises a further step (g) after step (e):

(g) providing the large scale perfusion culture of cells in suspension using the one or more culturing conditions used in step (b).

The large scale perfusion culture provided in step (g) is the large scale perfusion culture to be simulated by the method and uses the one or more culturing conditions of the large scale perfusion culture to be simulated which are used in step (b).

In a second aspects, the present invention provides a method for screening a culturing condition and/or a cell clone for a large scale perfusion culture of cells in suspension, comprising

(a) providing small scale cell cultures having a culture volume of 10% or less of the large scale perfusion culture;

(b) cultivating cells in the small scale cell cultures, wherein the small scale cell cultures are stirred;

(c) stopping the stirring of the small scale cell cultures;

(d) removing a part of the culture medium of the small scale cell cultures from the top part of the cell culture after at least partial sedimentation of the cells in the small scale cell cultures;

(e) adding fresh culture medium to the small scale cell cultures; and

(f) selecting the screened for culturing condition and/or cell clone which provides for a desired property and/or characteristic of the large scale perfusion culture;

wherein the individual small scale cell cultures differ in the culturing condition and/or the cell clone to be screened for; and wherein the cells are eukaryotic cells which grow in suspension.

In step (a), two or more small scale cell cultures are provided. In particular, at least 3, at least 4, at least 5, at least 8 or at least 10 small scale cell cultures are provided.

In specific embodiments, one or more, in particular all, of the culturing conditions which are not screened for are similar or identical between the small scale cell cultures. Suitable culturing conditions which can be screened for include in particular those selected from the group consisting of culture medium or specific culture medium ingredients, temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium.

In embodiments where the method is for screening a cell clone for a large scale perfusion culture, the different cell clones used in the different small scale cell cultures in particular are derived from the same parent cell. In cases where the cells recombinantly produce a biomolecule of interest, the different clones especially produce the same biomolecule, and in particular may be obtained by the same transfection experiment.

The desired property and/or characteristic of the large scale perfusion culture in particular may be selected from the group consisting of cell growth, cell viability and cell density. In embodiments where the cells produce a biomolecule of interest, in particular a glycoprotein, the property and/or characteristic of the large scale perfusion culture may further include the yield of the biomolecule of interest, the productivity of the cells, the stability of the production of the biomolecule of interest and the quality of the biomolecule of interest, including in particular one or more glycosylation characteristics of the biomolecule in case it is a glycoprotein. Exemplary glycosylation characteristics are described above.

In a certain embodiment, the method for screening a culturing condition and/or a cell clone for a large scale perfusion culture of cells in suspension comprises a further step (g) after step (f):

(g) providing the large scale perfusion culture of cells in suspension using the culturing condition and/or cell clone selected in step (f).

In certain embodiments, the further culturing conditions of the large scale perfusion culture provided in step (g) are similar or identical to the culturing conditions of the small scale cell cultures which are not screened for, where possible. Suitable culturing conditions which may be similar or identical between the large scale perfusion culture and the small scale cell culture include in particular those selected from the group consisting of culture medium or specific culture medium ingredients, temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium.

In certain embodiments, the method for screening a culturing condition and/or a cell clone for a large scale perfusion culture of cells in suspension comprises a further step (h):

(h) determining whether one or more of the small scale cell cultures have a property and/or characteristic which is desired for the large scale perfusion culture.

Step (h) may in particular be performed between steps (e) and (f). The property and/or characteristic determined in step (h) may be any one or more of the properties and/or characteristics described above with respect to step (f). Means and methods for determining the properties and/or characteristics are known to a person skilled in the art.

In embodiments wherein steps (b) to (e) are repeated one or more times, step (h) may also be performed during each cycle. Alternatively, step (h) may be performed once or several times after a certain number of cycles were done.

In certain embodiments, the large scale perfusion culture has a volume of at least 1 l, preferably at least 2 l, at least 5 l or at least 10 l. In specific embodiments, the large scale perfusion culture has a volume of at least 10 l or at least 100 l. Perfusion in this respect in particular means that a part of the culture medium of the cell culture is exchanged for fresh culture medium during the cultivation process. A perfusion culture hence differs from a batch culture. The perfusion in the large scale perfusion culture may be performed by any known means, and in particular is performed by using filtration or centrifugation systems. Suitable filtration systems are hollow fiber filters, especially used in alternating tangential flow (ATF) systems. For the centrifugation systems, in particular a continuous centrifuge can be used.

In specific embodiments, the small scale cell culture has a culture volume of 7.5% or less of the large scale perfusion culture, in particular a culture volume of 5% or less, 2.5% or less, 1.5% or less, or even 1% or less of the large scale perfusion culture. In certain embodiments, the small scale cell culture even has a culture volume of 0.5% or less, in particular 0.25% of the large scale perfusion culture.

The features and embodiments of the method for cultivating cells in suspension as described herein also apply likewise to the method for simulating a large scale perfusion culture of cells in suspension and the method for screening a culturing condition and/or a cell clone for a large scale perfusion culture of cells in suspension. A small scale cell culture may be used for simulating a large scale perfusion culture of cells in suspension, wherein the small scale cell culture is operated comprising the steps of

(a) providing a small scale cell culture having a culture volume of 10% or less of the large scale perfusion culture;

(b) cultivating cells in the small scale cell culture using one or more culturing conditions of the large scale perfusion culture to be simulated, wherein the small scale cell culture is stirred;

(c) stopping the stirring of the small scale cell culture;

(d) removing a part of the culture medium of the small scale cell culture after at least partial sedimentation of the cells in the small scale cell culture; and

(e) adding fresh culture medium to the small scale cell culture.

A small scale cell culture may be used for screening a culturing condition and/or a cell clone for a large scale perfusion culture of cells in suspension, wherein the small scale cell culture is operated comprising the steps of

(a) providing a small scale cell culture having a culture volume of 10% or less of the large scale perfusion culture;

(b) cultivating cells in the small scale cell culture, wherein the small scale cell culture is stirred;

(c) stopping the stirring of the small scale cell culture;

(d) removing a part of the culture medium of the small scale cell culture after at least partial sedimentation of the cells in the small scale cell culture; and

(e) adding fresh culture medium to the small scale cell culture.

In certain embodiments, more than one small scale cell culture is used and the individual small scale cell cultures differ in the culturing condition and/or the cell clone to be screened for.

The features and embodiments of the methods described herein also apply likewise to the uses of the small scale cell culture.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

Numeric ranges described herein are inclusive of the numbers defining the range. The headings provided herein are not limitations of the various aspects or embodiments of this invention which can be read by reference to the specification as a whole. According to one embodiment, subject matter described herein as comprising certain steps in the case of methods or as comprising certain ingredients in the case of compositions refers to subject matter consisting of the respective steps or ingredients. It is preferred to select and combine specific aspects and embodiments described herein and the specific subject-matter arising from a respective combination of specific embodiments also belongs to the present disclosure.

**FIGURES**

**Figure 1** shows the viable cell concentration (A+C) and the viability (B+D) of immortalized human blood cells of myeloid leukemia origin (A+B) and CHO cells (C+D) during sedimentation (up to 40 min) and after culture medium exchange and restart of stirring (value at 41 min).

**Figure 2** shows the effect of sedimentation on productivity (A) and glucose (B-C) / glutamine (D-E) metabolism for continuously stirred and discontinuously stirred antibody producing clone. Cells were cultivated for 11 hours. Stirring was stopped for sedimented cells (squares) but not for control cells (circles) (error bars are s.d., n=3).

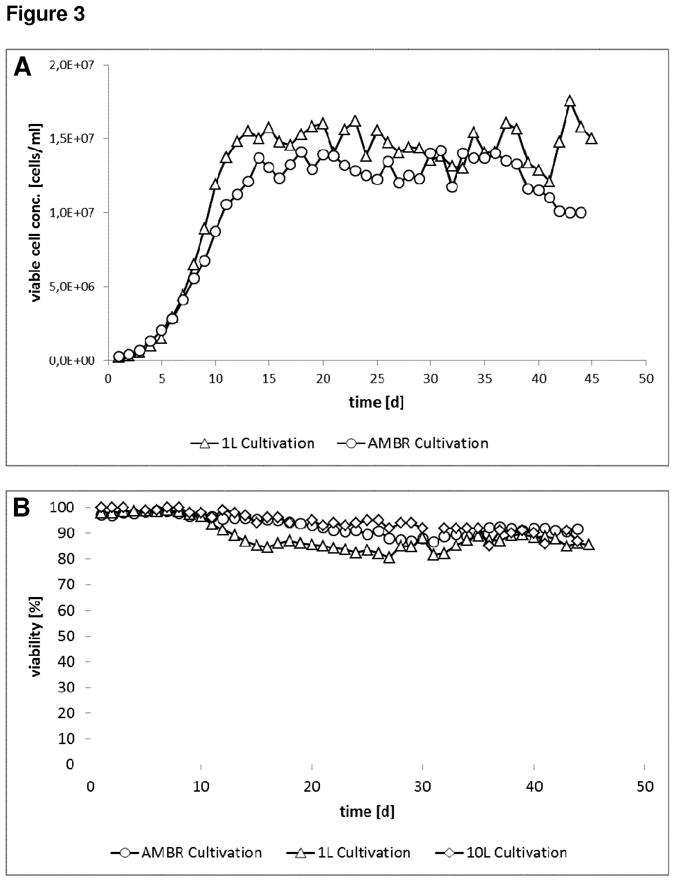

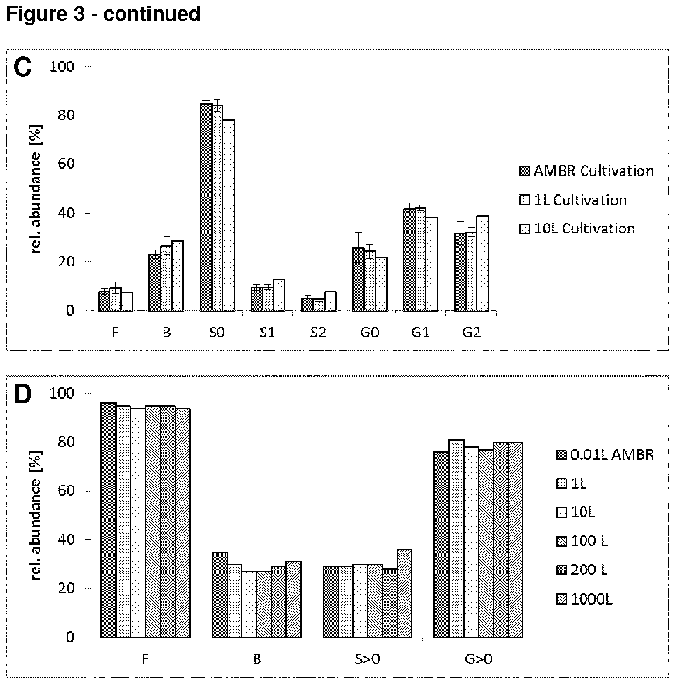

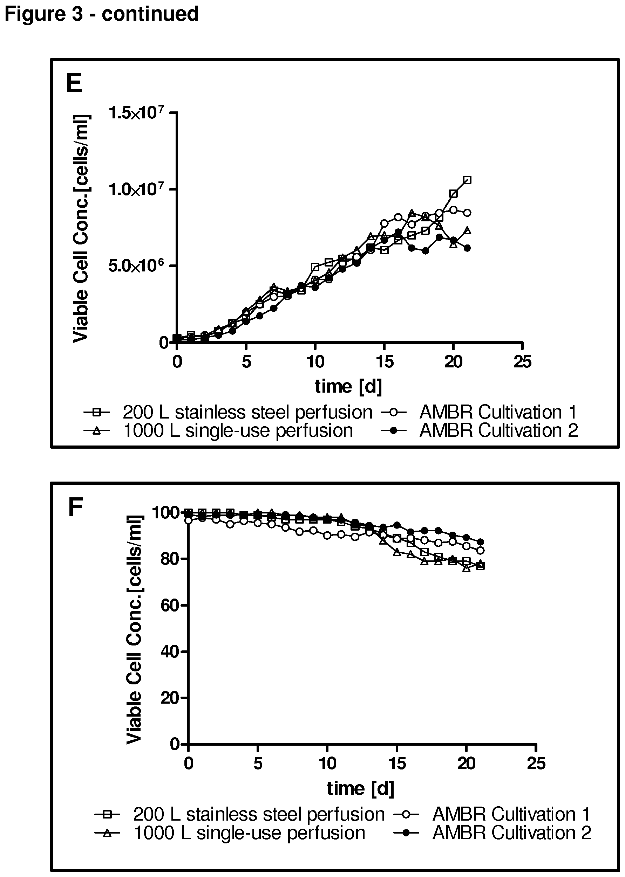

**Figure 3** shows the viable cell concentration (A), viability (B) and product glycosylation (C) of 12 mL sedimentation based ambr cultivation (5 runs), 1L perfusion culture (7 runs) and 10L single use bioreactor culture of clone 3 (GEX cell line) recombinantly producing IgG antibody. Shown are mean±SEM (A-B) and mean±SD (C). Culture conditions (DO, pH, temperature, media) are comparable for all scales. Product glycosylation of a different clone producing a different IgG antibody in 10 mL sedimentation based ambr cultivation and standard cultivations from 1 L to 1000 L is shown in (D). Furthermore, the viable cell concentration (E), and viability (F) of 12 mL sedimentation based ambr cultivation (2 runs), 200 L centritech perfusion culture in a stainless steel bioreactor and a 1000 L single use perfusion bioreactor culture of clone 4 (GEX cell line) recombinantly producing IgG antibody is shown. Culture conditions (DO, pH, temperature, media) are comparable for all scales.

**Figure 4** shows the degree of fucosylation for the IgG antibody produced by clone 1 (A) and clone 2 (B) for the 12 mL scale ambr culture in batch mode (white), the small scale ambr culture in perfusion mode (red) and the 1L scale culture (grey, black).

**Figure 5** shows cell concentration (A) and viability (B) of CHO cell cultures applying sedimentation based perfusion in the ambr system.

## EXAMPLES

**Example 1:** *Cultivation in microbioreactor system*

Microbioreactor cultivations are performed in the ambr™ (advanced mircobioreactor) system (TAP Biosystems/Sartorius). The ambr system is available as 24-way or 48-way. In this work only the 24-way is used which consists of two culture stations housing 12 vessels each. Each vessel is equipped with a pitched blade impeller with a diameter of 11 mm. pH and DO are measured by optical spots on the bottom of each vessel every 90 seconds. Each vessel is individually supplied with nitrogen (or air), oxygen (to maintain DO), carbon dioxide (to decrease pH) or a mix of these gases. Vessels are presterilized and available with or without sparge tube. For the cultivation of human myeloid leukemia derived cells (GEX cells) spargeless vessels are used. CHO DG44 cells are cultivated with or without sparge tube depending on the application. Cultivation temperature and stirring speed are set for each culture station (set of 12 vessels). All cell lines are cultivated at 37°C. GEX cells are stirred with 830 rpm up to 1000 rpm and CHO cells between 830 and 1300 rpm depending on DO concentration. pH is maintained by addition of 0.5 M NaOH (triggered below pH 6.9) and by supplying $CO_2$ (triggered above pH 7.3). Sampling and addition of media and feed is performed by a programmable liquid handler using single use tips with 1 mL or 5 mL maximum volume. The working volume of each vessel is 12 mL (10 mL minimum, 15 mL maximum). Samples for cell concentration and metabolite measurement are taken daily (between 250 and 450 $\mu$L depending on cell density). pH was calibrated every 3-4 days by offline measurements. To ensure sterile operation the entire system is placed under a biological safety cabinet. Vessels are inoculated with $2 \times 10^5$ cells/mL for GEX cells or $3 \times 10^5$ cells/mL for CHO cells. Cultivation was terminated when viability dropped below 65%. Antifoam for sparged vessels is supplied when needed.

**Example 2:** *Perfusion in microbioreactor system*

For perfusion mode cells are inoculated as described above. Perfusion is started with 12 mL and when glucose concentration drops below 2.5 g/L (usually after 72 to 120h). Cell retention is ensured by sedimentation. To allow the cells to settle stirring is stopped for the entire culture station. Ten minutes prior to stirring stop the pH control with NaOH is stopped to prevent addition of base directly before stirring stop. DO control is simultaneously stopped with stirring. Cells are allowed to settle for 30 to 45 minutes, preferably 40 minutes. After this time 3 mL of harvest is removed by the liquid handler from the top of the liquid and placed on 24-deep wells plates. Sample depth of the ambr system is altered to ensure that the harvest is taken from the top of the cultivation liquid (standard sample depth is set to take samples near the bottom of the vessel). The removal of the harvest is carried out by multiple pipetting operations for each vessel. After removal of harvest stirring is continued and the volume of the cultivation is restored to 12 mL by addition of fresh medium supplied in deep well plates by the liquid handler. DO and pH control is continued after addition of medium. All steps are carried out by the system autonomously (no user interaction). All steps taken together endure approximately 1 h. Per Step 3 mL (25% of the working volume) are exchanged. For a perfusion rate of one reactor volume ($V_r$/d) four steps are necessary per day. User interaction is limited to daily sampling, reloading of single use tips, removal of harvest and replenishing of feed media.

**Table 1: Steps carried out on the ambr system to enable perfusion operation**

| Order | Time course [min] | Command |
|---|---|---|
| 1 | 0 | Stop Background pH Addition |
| 2 | 10 | Stop Control DO pH |
| 3 | 10 | Stop Stirring |
| 4 | 50 | Sample Liquid From Culture Vessel |
| 5 | 50 | Start Stirring |
| 6 | 50 | Add Liquid To Culture Top Up |

(continued)

| Order | Time course [min] | Command |
|---|---|---|
| 7 | 50 | Start Control DO pH |
| 8 | 60 | Start Background pH Addition |

**Example 3:** *Determination of the settling time*

The following examples are performed with immortalized human blood cells of myeloid leukemia origin or with CHO cells. For analysis of the production of the cells and the product quality, the cells were transfected so as to recombinantly produce a glycosylated antibody.

To determine the optimal settling time, stirring is stopped and cell counts are taken before settling, after 10, 15, 20, 25, 30, 35 and 40 minutes after stirring stop and directly after continuation of stirring and addition of fresh media.

Separation efficiency (SE) is commonly used to express the performance of cell retention in perfusion culture and calculated as follows (with $c_{Reactor}$ being the concentration of cells in the culture medium in the bioreactor and $c_{Harvest}$ being the concentration of cells in the culture medium removed from the bioreactor after the settling time):

$$SE = \frac{c_{Reactor} - c_{Harvest}}{c_{Reactor}} \cdot 100$$

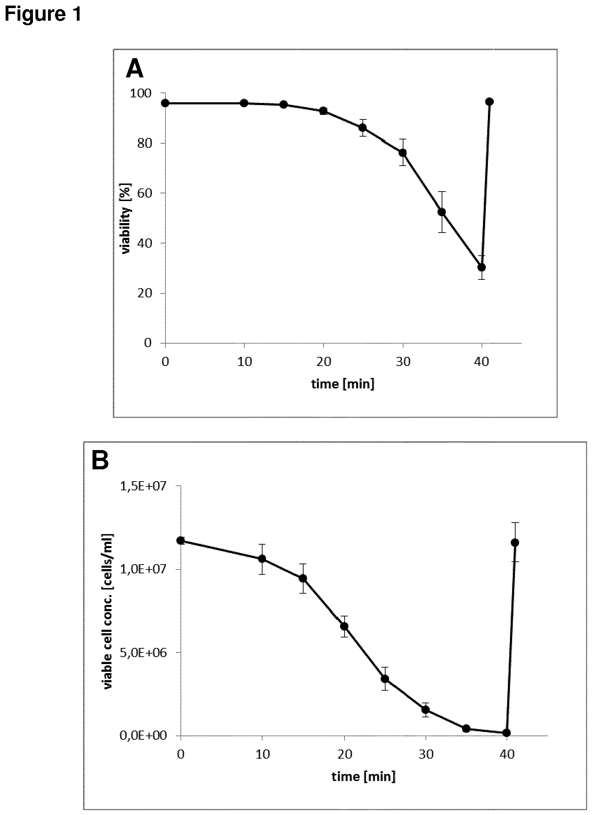

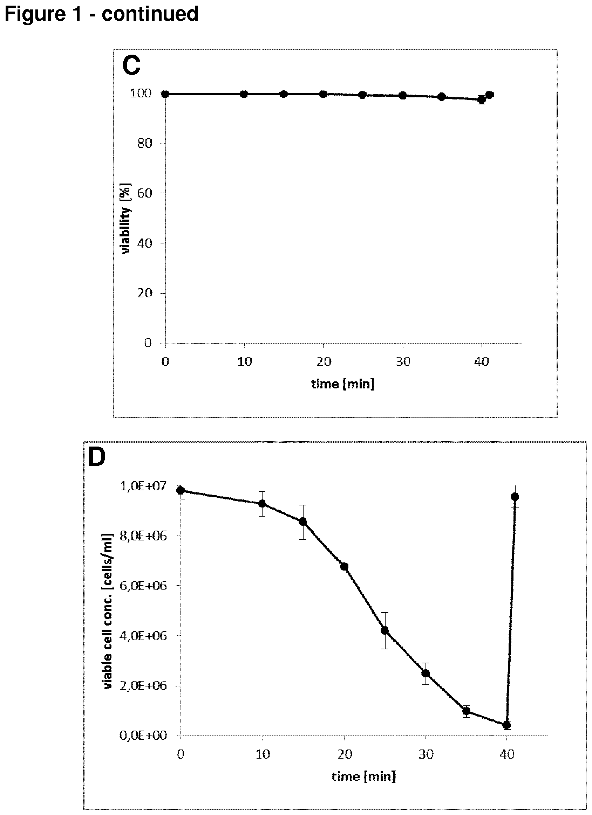

For both cell lines a sedimentation time of 40 minutes at an initial cell density of about $1 \times 10^7$ cells/mL is sufficient to achieve a separation efficiency of >95% meaning an almost cell free harvest. Even separation efficiencies below 90% are satisfactory and sometimes preferable to include an automatic cell bleeding. The degree of bleeding can be adjusted by the settling time (shorter settling time = higher bleeding rate). After continuation of stirring cell density is restored to the starting cell density showing that the perfusion step is operational without losing cells (see Figure 1A). Furthermore, also the cell viability is not affected by the sedimentation step (see Figure 1B).

To further study the effect of sedimentation on productivity and key metabolites an ambr run was started with typical process cell densities of $1 \times 10^7$ cell per ml. The control culture was continuously stirred whereas the sedimented vessels where discontinuously mixed with four breaks for 40 minutes of stirring within 110 minutes. Medium was not exchanged in both cell culture setups.

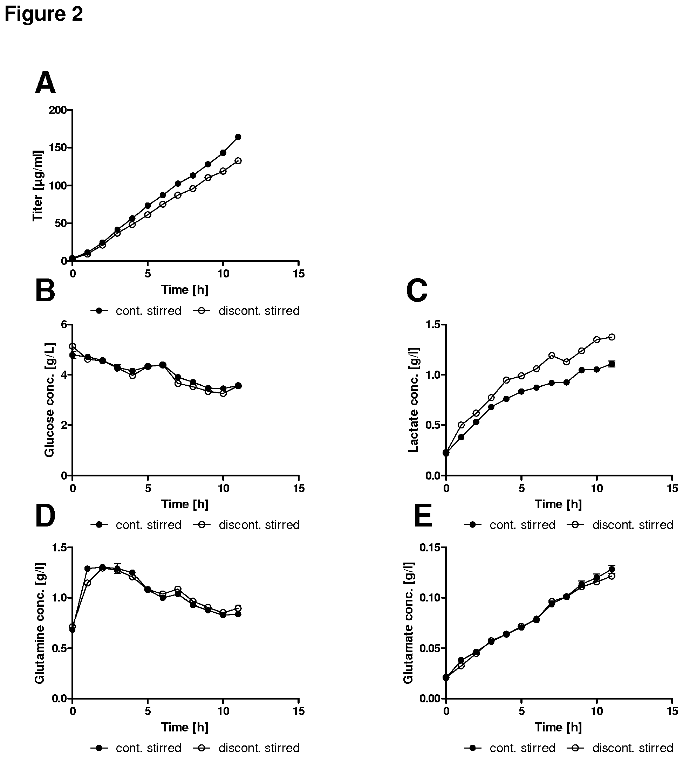

As seen in Figure 2 B, C and E glucose, glutamine and glutamate levels do not differ between the culture conditions as well as viable cell concentration and viability (data not shown). For titer and lactate levels differences can be observed. The regularly sedimented cells show higher lactate levels than continuously stirred cells. When stirring and gassing is stopped to initiate sedimentation oxygen transfer rapidly decreases leading to hypoxic conditions. This effect is even more drastic for accumulated cells at the bottom of the vessel where the local cell concentration is much higher than under well mixed conditions. Under hypoxic conditions cells rely more on anaerobic energy production which for mammalian cells with cancer origin means the conversion of glucose to lactate. Hypoxic conditions in CHO cells has been also shown to decrease protein productivity which agrees with the reduced IgG accumulation shown in Figure 2 A where the specific mAb production rate is decreased from $14.7 \pm 0.2$ μg/mL h to 12.1 $7 \pm 0.1$ μg/mL h.

**Example 4:** *Comparison of the cell growth and product quality*

Cell growth and product quality were compared between the microbioreactor ambr system as described in examples 1 and 2, above, and a 1 L cultivation in a perfusion bioreactor.

The 1 L cultivations were inoculated with $2.0 \times 10^5$ cells/mL. When glucose dropped below 2.5 g/L (usually day 4-5) continuous operation was enabled by feeding medium at a perfusion rate of 0.5 V/d. Perfusion was increased when glucose concentration remained below 2.5 g/L but at least every other day. Maximum perfusion rate was 2 reactor volumes a day. pH was controlled by addition of 0.5 M NaOH or sparging $CO_2$. Cultivation was terminated when viability dropped below 65% or when the intended run time was reached. Laboratory 1 L scale cultivations were carried out in Sartorius Biostat B-DCU 21. Dissolved oxygen and pH are measured by standard electrodes (Mettler Torledo InPro 6800 and Mettler-Torledo 405-DPAS-SC-K8S, respectively, Mettler Torledo, Switzerland). Agitation is done by a 3-blade segment impellers. Perfusion is performed using either an ATF2 module or a Centritech centrifuge. The ATF2 module has a 60 cm PES membrane (0.2 μm pore size and 0.15 m² membrane area, Spectrum, USA). Due to dead volume inside the ATF2 module, a membrane change goes along with a bleeding of 100-150 mL of cell suspension. The stand-alone continuous centrifuge Centritech Lab II or Lab III (successor of Lab II) (Berry Wehrmiller, Pneumatic scale) is used in intermittent mode working discontinuously. This is recommended for small scale volumes. The wait time between the cycles is adjusted to control the perfusion rate. At the end of each running cycle, all tubes are flushed with supernatant in order to back-flush remaining cells into the bioreactor.

Growth profile of 1 L or 10 L cultivations in a perfusion bioreactor and small scale cultivation in the ambr system are highly comparable. The cultures reach the same maximum cell density. Viability of the 1 L perfusion culture is a bit lower than for the sedimentation based perfusion in the small scale ambr system or the 10 L large scale culture, but comparable. Product glycosylation patterns are highly comparable between the small scale ambr cultivation and large scale cultures even up to a volume of 1000 L (mean values obtained from different runs with non-relevant changes in process control) (see Figure 3). Further quality attributes are listed in Table 2:

**Table 2: Additional product quality analysis.**

| Assay / parameter | Result |
|---|---|
| SEC | 0,00% multimer / 0.26% dimer / 0.27% fragment Complies with specification |
| SDS-PAGE | Clear bands for heavy and light chains at correct MW |
| IEF | Slightly weaker basic bands |
| WCX | Same peak pattern as for standard |
| Antigen ELISA | Complies with specification |
| FcγRIIIa AlphaScreen | 1.01 potency |
| Oxidation (M254|M430) | Complies with specification |
| Deamidation (N288|N317|N386/391 |N423/436) | Complies with specification |
| Lysine-clipping | 98.7% (controls: 89,7%190,9%185.1%) |
| ADCC | 0.96 potency |

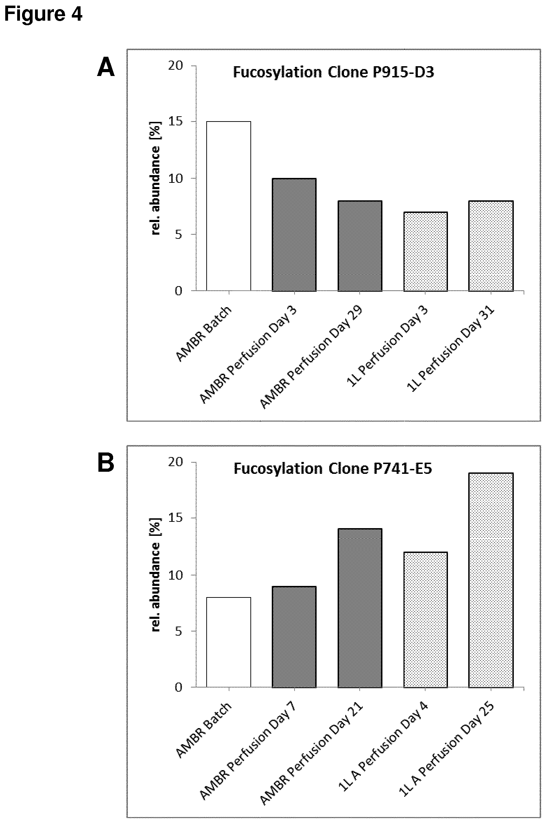

Figure 4 shows that the small scale ambr system in perfusion mode is much better suited to predict changes in glycosylation pattern during the course of bigger scale cultivations than the ambr system in batch mode. Especially clone P741-E5 shows an increase of fucosylation with time (grey bars in B) when comparing early harvests (perfusion day 4) to late harvests (perfusion day 25). An increase in fucosylation is also observed in the ambr perfusion but not in batch mode. Clone P915-D3 shows high fucosylation in batch mode but low fucosylation in ambr and 1L scale perfusion.

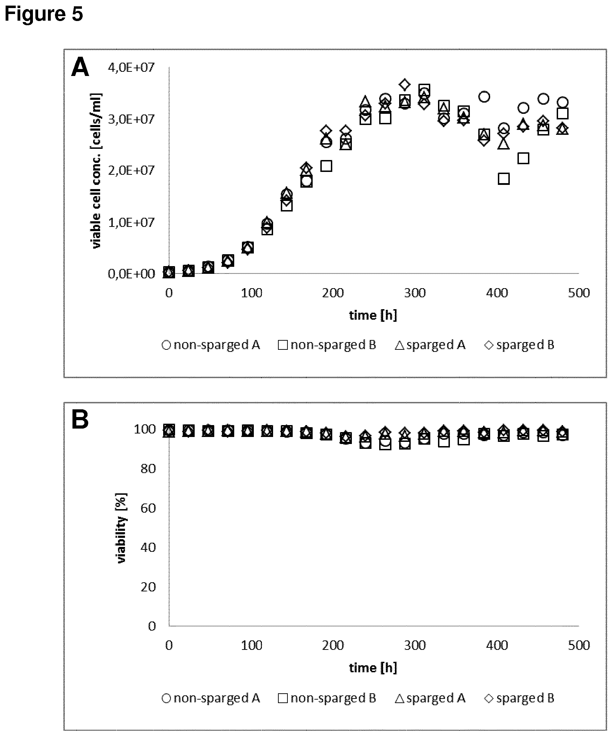

CHO DG44 cells show good and consistent growth up to 3.5 x 107 cells/mL in sedimentation based perfusion in the small scale ambr system independent of gas supply (sparged or non-sparged). Viability remains high during the entire culture (>90%) of 21 days (see Figure 5).

Glycosylation of monoclonal antibody produced at 1L and 12mL scale suing CHO DG44 derived cells is highly comparable. Galactosylation for ambr based perfusion is slightly decreased for the day ten harvest.

**Table 3: Glycan structure of monoclonal antibody produced in CHO DG44 at 1L and 12mL scale.**

| Scale | Cell Retention | Perfusion day | F | B | S>0 | G>0 |
|---|---|---|---|---|---|---|
| 1L | Centritech | 2 | 98 | 1 | 2 | 36 |
| 1L | Centritech | 10 | 97 | 1 | 5 | 33 |
| AMBR (12mL) | Sedimentation | 2 | 97 | 0 | 2 | 37 |
| AMBR (12mL) | Sedimentation | 10 | 95 | 0 | 2 | 28 |

F: fucosylated N-glycans; B: bisecting N-acetylglucosamine; S>0: sialylated N-glycans; G>0: galactosylated N-glycans. Relative abundances of glycan structures are related to the total amount of N-glycans.

**Example 5:** *Design of experiment (DoE) study*

To further prove the benefits of the here developed system, DoE studies are performed. As DoE factors the addition of a chemical chaperone in a concentration range of 0.5% to 1.5% (CC5), supplementation with GlycoMix with 0.5 to 1.5 times the standard concentration and the process pH (6.8 - 7,2) are used. A CCF (Central Composite Face) design with 4 center points and 14 design runs is chosen. Mean viability, integral viable cell density (IVCD), and total product per run are used as responses. The summary of the model fit is depicted in Table 4.

**Table 4: Summary of DoE fit**

| Response | $R^2$ | $Q^2$ | Validity | Reproducibility |
|----------|-------|-------|----------|-----------------|
| Product | 0.949 | 0.831 | 0.758 | 0.950 |
| IVCD | 0.972 | 0.845 | 0.950 | 0.930 |
| Viability | 0.928 | 0.749 | 0.975 | 0.785 |

$R^2$ values for all responses are high and close to 1 indicating a good model fit. The future prediction precision $Q^2$ is also high around 0.8 for all three responses. Good models show $Q^2$ values of greater than 0.5. Validity (should be greater than 0.25) and reproducibility (should be greater than 0.5) are acceptable as well.

**Identification of the deposited biological material**

The cell lines DSM ACC 2606 and DSM ACC 2605 were deposited at the DSMZ - Deutsche Sammlung von Mikroorganismen und Zellkulturen GmbH, Mascheroder Weg 1b, 38124 Braunschweig (DE) by Nemod Biotherapeutics GmbH & Co. KG, Robert-Rossle-Str. 10, 13125 Berlin (DE) on August 14, 2003. Glycotope is entitled to refer to these biological materials since they were in the meantime assigned from Nemod Biotherapeutics GmbH & Co. KG to Glycotope GmbH.

The cell lines DSM ACC 2806, DSM ACC 2807, DSM ACC 2856, DSM ACC 2858 and DSM ACC 3078 were deposited at the DSMZ - Deutsche Sammlung von Mikroorganismen und Zellkulturen GmbH, Inhoffenstraße 7B, 38124 Braunschweig (DE) by Glycotope GmbH, Robert-Rossle-Str. 10, 13125 Berlin (DE) on the dates indicated in the following table.

| Name of the Cell Line | Accession Number | Depositor | Date of Deposition |
|-----------------------|------------------|-----------|--------------------|
| NM-F9 | DSM ACC 2606 | Nemod Biotherapeutics | August 14, 2003 |
| NM-D4 | DSM ACC 2605 | Nemod Biotherapeutics | August 14, 2003 |
| NM-H9D8 | DSM ACC 2806 | Glycotope GmbH | September 15, 2006 |
| NM-H9D8-E6 | DSM ACC 2807 | Glycotope GmbH | October 5, 2006 |
| NM-H9D8-E6Q12 | DSM ACC 2856 | Glycotope GmbH | August 8, 2007 |
| GT-2x | DSM ACC 2858 | Glycotope GmbH | September 7, 2007 |
| GT-5s | DSM ACC 3078 | Glycotope GmbH | July 28, 2010 |

**Claims**

1. A method for simulating a large scale perfusion culture of cells in suspension, comprising

   (a) providing a small scale cell culture having a culture volume of 10% or less of the large scale perfusion culture;
   (b) cultivating cells in the small scale cell culture using one or more culturing conditions of the large scale perfusion culture to be simulated, wherein the small scale cell culture is stirred;
   (c) stopping the stirring of the small scale cell culture;
   (d) removing a part of the culture medium of the small scale cell culture from the top part of the cell culture after at least partial sedimentation of the cells in the small scale cell culture; and
   (e) adding fresh culture medium to the small scale cell culture;

   wherein the cells are eukaryotic cells which grow in suspension.

2. The method according to claim 1, wherein the one or more culturing conditions of the large scale perfusion culture to be simulated which are used for cultivating cells in the small scale cell culture are selected from the group consisting of culture medium, temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium.

3. A method for screening a culturing condition and/or a cell clone for a large scale perfusion culture of cells in suspension, comprising

(a) providing small scale cell cultures having a culture volume of 10% or less of the large scale perfusion culture;
(b) cultivating cells in the small scale cell cultures, wherein the small scale cell cultures are stirred;
(c) stopping the stirring of the small scale cell cultures;
(d) removing a part of the culture medium of the small scale cell cultures from the top part of the cell culture after at least partial sedimentation of the cells in the small scale cell cultures;
(e) adding fresh culture medium to the small scale cell cultures; and
(f) selecting the screened for culturing condition and/or cell clone which provides for a desired property and/or characteristic of the large scale perfusion culture;

wherein the individual small scale cell cultures differ in the culturing condition and/or the cell clone to be screened for; and wherein the cells are eukaryotic cells which grow in suspension.

4. The method according to claim 3, wherein the culturing conditions to be screened for are selected from the group consisting of culture medium, temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium.

5. The method according to claim 3 or 4, wherein those culturing conditions and/or cell clones are selected in the screening which provide for a high cell viability, a high and/or stable cell density, a high and/or stable yield of a biomolecule of interest produced by the cells in the cell culture, and a desired property of a biomolecule of interest produced by the cells in the cell culture.

6. The method according to any one of claims 1 to 5, wherein the large scale perfusion culture has a volume of at least 1 l, and/or wherein the small scale cell culture has a volume of 50 ml or less.

7. The method according to any one of claims 1 to 6, wherein after stopping the stirring in step (c) sedimentation of the cells takes place for at least 35 min before removing a part of the culture medium in step (d).

8. The method according to any one of claims 1 to 6, wherein after stopping the stirring in step (c) sedimentation of the cells takes place for 30 min or less before removing a part of the culture medium in step (d), and wherein in step (d) also a part of the cells in the cells culture is removed to effect cell bleeding.

9. The method according to any one of claims 1 to 8, wherein between 15% and 50% of the culture medium is removed in step (d).

10. The method according to any one of claims 1 to 9, wherein the amount of fresh culture medium added in step (e) is in essence identical to the amount of culture medium removed in step (d).

11. The method according to any one of claims 1 to 10, wherein the pH value and/or the oxygen concentration in the culture medium are controlled during cultivation of the cells; and wherein the control of the pH value and/or the oxygen concentration is stopped at least 5 min before stopping the stirring of the cell culture in step (c).

12. The method according to any one of claims 1 to 11, wherein steps (b) to (e) are repeated one or more times, and wherein after step (e) the cells are cultured under stirring for at least 1 h before the stirring is stopped in step (c) of the next cycle.

13. The method according to any one of claims 1 to 12, wherein the cells to be cultured are immortalized human blood cells such as immortalized human cells of myeloid leukemia origin, or CHO cells.

14. The method according to any one of claims 1 to 13, wherein the cells recombinantly produce a biomolecule of interest, preferably a glycoprotein.

**Patentansprüche**

1. Verfahren zur Simulation einer großmaßstäbigen Perfusionskultur von Zellen in Suspension, umfassend

(a) Bereitstellen einer kleinmaßstäbigen Zellkultur mit einem Kulturvolumen von 10% oder weniger der großmaßstäbigen Perfusionskultur;
(b) Kultivieren von Zellen in der kleinmaßstäbigen Zellkultur unter Verwendung einer oder mehrerer Kulturbedingungen der zu simulierenden großmaßstäbigen Perfusionskultur, wobei die kleinmaßstäbige Zellkultur gerührt wird;
(c) Stoppen des Rührens der kleinmaßstäbigen Zellkultur;
(d) Entfernen eines Teils des Kulturmediums der kleinmaßstäbigen Zellkultur aus dem oberen Teil der Zellkultur nach zumindest teilweiser Sedimentation der Zellen in der kleinmaßstäbigen Zellkultur; und
(e) Zugeben von frischem Kulturmedium zur kleinmaßstäbigen Zellkultur;

wobei die Zellen eukaryotische Zellen sind, die in Suspension wachsen.

2. Das Verfahren nach Anspruch 1, **dadurch gekennzeichnet, dass** die eine oder mehrere Kulturbedingungen der zu simulierenden großmaßstäbigen Perfusionskultur, die für die Kultur von Zellen in der kleinmaßstäbigen Zellkultur verwendet werden, ausgewählt sind aus der Gruppe bestehend aus Kulturmedium, Temperatur, pH-Wert, Sauerstoffkonzentration, Zelldichte, Zugabe von Zellnährstoffen einschließlich des Zeitpunkts der Zugabe und der Zielkonzentration der Nährstoffe im Kulturmedium, Kulturdauer, spezifischer Leistungsaufnahme, Rate der Gaszufuhr, Rate der Perfusion und Osmolarität des Kulturmediums.

3. Verfahren zum Screening einer Kulturbedingung und/oder eines Zellklons für eine großmaßstäbige Perfusionskultur von Zellen in Suspension, umfassend

(a) Bereitstellen von kleinmaßstäbigen Zellkulturen mit einem Kulturvolumen von 10% oder weniger der großmaßstäbigen Perfusionskultur;
(b) Kultivieren von Zellen in den kleinmaßstäbigen Zellkulturen, wobei die kleinmaßstäbigen Zellkulturen gerührt werden;
(c) Stoppen des Rührens der kleinmaßstäbigen Zellkulturen;
(d) Entfernen eines Teils des Kulturmediums der kleinmaßstäbigen Zellkulturen aus dem oberen Teil der Zellkultur nach zumindest teilweiser Sedimentation der Zellen in den kleinmaßstäbigen Zellkulturen;
(e) Zugeben von frischem Kulturmedium zu den kleinmaßstäbigen Zellkulturen; und
(f) Auswählen der gescreenten Kulturbedingung und/oder des Zellklons, die/der für eine gewünschte Eigenschaft und/oder ein gewünschtes Merkmal der großmaßstäbigen Perfusionskultur sorgen;

wobei sich die einzelnen kleinmaßstäbigen Zellkulturen in der Kulturbedingung und/oder dem Zellklon unterscheiden, auf die gescreent werden soll, und wobei die Zellen eukaryotische Zellen sind, die in Suspension wachsen.

4. Das Verfahren nach Anspruch 3, **dadurch gekennzeichnet, dass** die Kulturbedingungen, auf die gescreent werden soll, ausgewählt sind aus der Gruppe bestehend aus Kulturmedium, Temperatur, pH-Wert, Sauerstoffkonzentration, Zelldichte, Zugabe von Zellnährstoffen einschließlich des Zeitpunkts der Zugabe und der Zielkonzentration der Nährstoffe im Kulturmedium, Kulturdauer, spezifischer Leistungsaufnahme, Rate der Gaszufuhr, Rate der Perfusion und Osmolarität des Kulturmediums.

5. Das Verfahren nach Anspruch 3 oder 4, **dadurch gekennzeichnet, dass** bei dem Screening diejenigen Kulturbedingungen und/oder Zellklone ausgewählt werden, die für eine hohe Zellviabilität, eine hohe und/oder stabile Zelldichte, eine hohe und/oder stabile Ausbeute eines Biomoleküls von Interesse, das von den Zellen in der Zellkultur produziert wird, und für eine gewünschte Eigenschaft eines Biomoleküls von Interesse, das von den Zellen in der Zellkultur produziert wird, sorgen.

6. Das Verfahren nach einem der Ansprüche 1 bis 5, **dadurch gekennzeichnet, dass** die großmaßstäbige Perfusionskultur ein Volumen von mindestens 1 l aufweist und/oder die kleinmaßstäbige Zellkultur ein Volumen von 50 ml oder weniger aufweist.

7. Das Verfahren nach einem der Ansprüche 1 bis 6, **dadurch gekennzeichnet, dass** nach dem Stoppen des Rührens in Schritt (c) eine Sedimentation der Zellen für mindestens 35 min erfolgt, bevor ein Teil des Kulturmediums in Schritt (d) entfernt wird.

8. Das Verfahren nach einem der Ansprüche 1 bis 6, **dadurch gekennzeichnet, dass** nach dem Stoppen des Rührens in Schritt (c) eine Sedimentation der Zellen für 30 min oder weniger stattfindet, bevor ein Teil des Kulturmediums in Schritt (d) entfernt wird, und in Schritt (d) auch ein Teil der Zellen in der Zellkultur entfernt wird, um ein Zellbleeding zu bewirken.

9. Das Verfahren nach einem der Ansprüche 1 bis 8, **dadurch gekennzeichnet, dass** in Schritt (d) zwischen 15% und 50% des Kulturmediums entfernt werden.

10. Das Verfahren nach einem der Ansprüche 1 bis 9, **dadurch gekennzeichnet, dass** die in Schritt (e) zugegebene Menge an frischem Kulturmedium im Wesentlichen identisch ist mit der in Schritt (d) entnommenen Menge an Kulturmedium.

11. Das Verfahren nach einem der Ansprüche 1 bis 10, **dadurch gekennzeichnet, dass** der pH-Wert und/oder die Sauerstoffkonzentration im Kulturmedium während der Kultur der Zellen geregelt werden und die Regelung des pH-Wertes und/oder der Sauerstoffkonzentration mindestens 5 min vor dem Stoppen des Rührens der Zellkultur in Schritt (c) gestoppt wird.

12. Das Verfahren nach einem der Ansprüche 1 bis 11, **dadurch gekennzeichnet, dass** die Schritte (b) bis (e) ein oder mehrere Male wiederholt werden und nach Schritt (e) die Zellen mindestens 1 h lang unter Rühren kultiviert werden, bevor das Rühren in Schritt (c) des nächsten Zyklus gestoppt wird.

13. Das Verfahren nach einem der Ansprüche 1 bis 12, **dadurch gekennzeichnet, dass** die zu kultivierenden Zellen immortalisierte humane Blutzellen, wie immortalisierte humane Zellen myeloischen Leukämie-Ursprungs, oder CHO-Zellen sind.

14. Das Verfahren nach einem der Ansprüche 1 bis 13, **dadurch gekennzeichnet, dass** die Zellen rekombinant ein Biomolekül von Interesse produzieren, bevorzugt ein Glykoprotein.

**Revendications**

1. Procédé pour simuler une culture de cellules par perfusion à grande échelle en suspension, comprenant :

   (a) la constitution d'une culture de cellules à petite échelle présentant un volume de culture qui est égal à 10 % ou moins du volume de la culture de cellules par perfusion à grande échelle ;
   (b) la mise en culture de cellules dans la culture de cellules à petite échelle en utilisant une ou plusieurs condition(s) de mise en culture de la culture de cellules par perfusion à grande échelle qui est destinée à être simulée, dans lequel la culture de cellules à petite échelle est agitée ;
   (c) l'arrêt de l'agitation de la culture de cellules à petite échelle ;
   (d) la suppression d'une partie du milieu de culture de la culture de cellules à petite échelle au niveau de la partie sommitale de la culture de cellules après une sédimentation au moins partielle des cellules dans la culture de cellules à petite échelle ; et
   (e) l'ajout de milieu de culture frais à la culture de cellules à petite échelle ;

   dans lequel les cellules sont des cellules eucaryotes qui croissent en suspension.

2. Procédé selon la revendication 1, dans lequel les une ou plusieurs conditions de mise en culture de la culture de cellules par perfusion à grande échelle qui est destinée à être simulée, lesquelles conditions de mise en culture sont utilisées pour mettre en culture des cellules dans la culture de cellules à petite échelle, sont sélectionnées parmi le groupe qui est constitué par le milieu de culture, la température, la valeur de pH, la concentration en oxygène, la densité des cellules, l'ajout de nutriments de cellules, incluant le temps d'ajout et la concentration cible des nutriments dans le milieu de culture, le temps de mise en culture, l'entrée en termes d'alimentation en énergie électrique spécifique, le débit de l'alimentation en gaz, la vitesse de perfusion et l'osmolarité du milieu de culture.

3. Procédé pour déterminer une condition de mise en culture et/ou un clone cellulaire pour une culture de cellules par perfusion à grande échelle en suspension, comprenant :

(a) la constitution de cultures de cellules à petite échelle présentant un volume de culture qui est égal à 10 % ou moins du volume de la culture de cellules par perfusion à grande échelle ;

(b) la mise en culture de cellules dans les cultures de cellules à petite échelle, dans lequel les cultures de cellules à petite échelle sont agitées ;

(c) l'arrêt de l'agitation des cultures de cellules à petite échelle ;

(d) la suppression d'une partie du milieu de culture des cultures de cellules à petite échelle au niveau de la partie sommitale des cultures de cellules après une sédimentation au moins partielle des cellules dans les cultures de cellules à petite échelle ; et

(e) l'ajout de milieu de culture frais aux cultures de cellules à petite échelle; et

(f) la sélection de la condition de mise en culture et/ou du clone cellulaire déterminé(e)(s) qui assure(nt) une propriété et/ou une caractéristique souhaitée(s) de la culture de cellules par perfusion à grande échelle ;

dans lequel les cultures de cellules à petite échelle individuelles diffèrent en termes de condition de mise en culture et/ou de clone cellulaire qui sont destinées à être déterminé(e)(s) ; et dans lequel les cellules sont des cellules eucaryotes qui croissent en suspension.

4. Procédé selon la revendication 3, dans lequel les conditions de mise en culture qui sont destinées à être déterminées sont sélectionnées parmi le groupe qui est constitué par le milieu de culture, la température, la valeur de pH, la concentration en oxygène, la densité des cellules, l'ajout de nutriments de cellules, incluant le temps d'ajout et la concentration cible des nutriments dans le milieu de culture, le temps de mise en culture, l'entrée en termes d'alimentation en énergie électrique spécifique, le débit de l'alimentation en gaz, la vitesse de perfusion et l'osmolarité du milieu de culture.

5. Procédé selon la revendication 3 ou 4, dans lequel les conditions de mise en culture et/ou les clones cellulaires qui sont sélectionné(e)s lors de la détermination sont tel(le)s qu'elles/ils assurent une viabilité élevée des cellules, une densité élevée et/ou stable des cellules, un rendement biomoléculaire élevé et/ou stable d'une biomolécule d'intérêt produite au moyen des cellules dans la culture de cellules et une propriété souhaitée d'une biomolécule d'intérêt produite au moyen des cellules dans la culture de cellules.

6. Procédé selon l'une quelconque des revendications 1 à 5, dans lequel la culture de cellules par perfusion à grande échelle présente un volume d'au moins 1 l, et/ou dans lequel la culture de cellules à petite échelle présente un volume de 50 ml ou moins.

7. Procédé selon l'une quelconque des revendications 1 à 6, dans lequel, après l'arrêt de l'agitation au niveau de l'étape (c), la sédimentation des cellules est réalisée pendant au moins 35 minutes avant la suppression d'une partie du milieu de culture au niveau de l'étape (d).

8. Procédé selon l'une quelconque des revendications 1 à 6, dans lequel, après l'arrêt de l'agitation au niveau de l'étape (c), la sédimentation des cellules est réalisée pendant 30 minutes ou moins avant la suppression d'une partie du milieu de culture au niveau de l'étape (d), et dans lequel, au niveau de l'étape (d), également une partie des cellules dans la culture de cellules est supprimée afin d'évacuer des cellules.

9. Procédé selon l'une quelconque des revendications 1 à 8, dans lequel une fraction entre 15 % et 50 % du milieu de culture est supprimée au niveau de l'étape (d).

10. Procédé selon l'une quelconque des revendications 1 à 9, dans lequel la quantité de milieu de culture frais qui est ajoutée au niveau de l'étape (e) est par essence identique à la quantité de milieu de culture qui est supprimée au niveau de l'étape (d).

11. Procédé selon l'une quelconque des revendications 1 à 10, dans lequel la valeur de pH et/ou la concentration en oxygène dans le milieu de culture sont/est contrôlée(s) pendant la culture des cellules ; et dans lequel le contrôle de la valeur de pH et/ou de la concentration en oxygène est arrêté au moins 5 minutes avant l'arrêt de l'agitation de la culture de cellules au niveau de l'étape (c).

12. Procédé selon l'une quelconque des revendications 1 à 11, dans lequel les étapes (b) à (e) sont répétées une ou plusieurs fois, et dans lequel, après l'étape (e), les cellules sont mises en culture sous agitation pendant au moins 1 heure avant que l'agitation ne soit arrêtée au niveau de l'étape (c) du cycle suivant.

**13.** Procédé selon l'une quelconque des revendications 1 à 12, dans lequel les cellules qui sont destinées à être mises en culture sont des cellules de sang humain immortalisées telles que des cellules humaines immortalisées dont l'origine est une leucémie myéloïde, ou des cellules CHO.

**14.** Procédé selon l'une quelconque des revendications 1 à 13, dans lequel les cellules produisent par recombinaison une biomolécule d'intérêt, de préférence une glycoprotéine.

## Figure 1

# Figure 1 - continued

# Figure 2

## Figure 3

**Figure 3 - continued**

# Figure 3 - continued

# Figure 4

A Fucosylation Clone P915-D3

B Fucosylation Clone P741-E5

## Figure 5

**EP 3 303 557 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012085162 A1

- EP 0599651 A2

**Non-patent literature cited in the description**

- **HSU et al.** *Cytotechnology,* 2012, vol. 64, 667-678
- **SHIMONI et al.** *BioProcess Int,* 2014, vol. 12, 54-61
- **VOISARD et al.** *Biotechol Bioeng,* 2003, vol. 82, 751-765

- **WOODSIDE et al.** *Cytotechnology,* 1998, vol. 28, 163-175
- **BLECKWENN et al.** *Current Protocols in Immunology,* 2004, A.1U.1-A.1U.44